# EUROPEAN PATENT SPECIFICATION

(11) **EP 1 991 201 B1**
(45) Date of publication and mention of the grant of the patent: **28.03.2018**
(21) Application number: 07703355.3
(22) Date of filing: 08.02.2007
(51) Int. Cl.: A61K 9/12, A61K 9/72, A61K 31/535, A61K 31/496, A61K 31/4709

(54) **NEBULISED ANTIBIOTICS FOR INHALATION THERAPY**
VERNEBELTE ANTIBIOTIKA FÜR DIE INHALATIONSTHERAPIE
ANTIBIOTIQUES NÉBULISÉS POUR THÉRAPIE PAR INHALATION

(30) Priority: 10.02.2006 EP 06002734
(43) Date of publication of application: 19.11.2008
(73) Proprietor: PARI Pharma GmbH, 82319 Starnberg (DE)
(72) Inventor: KELLER, Manfred, 81379 München (DE); AKKAR, Aslihan, 80805 München (DE)
(74) Representative: Beckmann, Claus
(86) International application number: PCT/EP2007/001080
(87) International publication number: WO 2007/090646

(56) References cited:
- WO-A-2004/089418
- WO-A-2004/110493
- WO-A1-97/23217
- US-A1- 2003 186 894
- US-A1- 2004 097 512
- US-A1- 2004 204 399
- US-A1- 2006 276 473
- WEBER A ET AL: "EFFECT OF NEBULIZER TYPE AND ANTIBIOTIC CONCENTRATION ON DEVICE PERFORMANCE" PEDIATRIC PULMONOLOGY, JOHN WILEY, NEW YORK, NY, US, vol. 23, no. 4, April 1997 (1997-04), pages 249-260, XP001118136 ISSN: 8755-6863

## Description

### FIELD OF THE INVENTION

The invention relates to pharmaceutical aerosols which are useful for the prevention or treatment of infectious diseases of the airways, such as the lungs, the bronchi, or the sinunasal cavities. It is also related to solid or liquid pharmaceutical compositions and kits for preparing such aerosols.

### BACKGROUND OF THE INVENTION

The delivery of therapeutic compounds to the bronchi and lungs has been used primarily for the local treatment of diseases and conditions of the respiratory system, such as asthma and bronchitis. More recently, the pulmonary administration of systemic drugs, such as insulin, has been proposed and actively pursued in product development programs, utilising the large surface area of the lungs for absorption.

In general, drug substances can be delivered to the respiratory system as aerosolised dry powders or liquids, the liquids representing either solutions or dispersions, such as drug suspensions. Various devices have been developed to convert a liquid or solid composition into an aerosol and to enable inhalation. One of the most important requirements for any such device is that it is capable of achieving a particles size of the aerosol which will allow deposition at the target site, i.e. the designated site of action or of absorption. Depending on whether the drug should be delivered to the bronchi or to the deep lungs, the optimal droplet or particle size for typical formulations may vary from about 10 microns down to below one micron; larger particles may be useful if their density is very low.

Metered-dose inhalers deliver a measured dose of the drug in the form of a suspension of small liquid or solid particles, which is dispensed from the inhaler by a propellant under pressure. Such inhalers are placed into the mouth and activated to release drug as the individual takes a breath. This requires a certain amount of coordination and may therefore be unsuitable for children.

Spacers, or spacing devices, which are available for use with some aerosol inhalers, extend the space between the inhaler and the mouth. This reduces the speed at which the aerosol travels to the back of the mouth, allowing more time for the propellant to evaporate and therefore reducing the impact of the propellant on the back of the mouth-which can cause irritation-and enabling a higher proportion of the particles of the drug to be inhaled. There is also less need to coordinate breathing in with activation of the inhaler. Breath-activated inhalers deliver the drug, in the form of an aerosol or a dry powder, only when the user places his mouth over the outlet and breathes in. This obviates the need to coordinate breathing in with depressing the dispenser. The dose of drug will still be measured or metered, and is not dependent on the size of breath taken.

Dry powder inhalers, on the other hand, are loaded with portions of the drug substance in form of a powder formulation. The unit doses may be accommodated in small capsules. As the inhaler is activated by taking a breath, a capsule is punctured and a type of fan mechanism disperses the powder so that it can be inhaled, as e.g. in the commercially available devices known as "Spinhaler" and "Rotahaler". Another device known as "Turbohalers" is fitted with cartridges that contain measured doses of the powder formulation.

Aqueous-based solutions and suspensions are usually inhaled with nebulisers. Various types of nebulisers are commercially available or presently being developed. A traditional type is the jet nebulizer, which is still being used extensively. More recently, ultrasonic and vibrating membrane-type nebulizers were developed.

While traditional inhalation therapies were primarily directed to the prevention and treatment of allergic and inflammatory diseases and conditions of the respiratory system including asthma and obstructive bronchitis, novel therapeutical approaches have been developed more recently. For instance, the local treatment of pulmonary infections with antibiotics has been suggested and, with tobramycin being the first antibiotic approved for this use, successfully introduced to the therapy of certain severe or even life-threatening types of infection. Tobramycin, which is supplied as Tobi™, is a sterile, clear, slightly yellow, non-pyrogenic, aqueous solution with the pH and salinity adjusted specifically for administration by a compressed air driven reusable nebuliser. It is approved for the treatment of cystic fibrosis patients infected with Pseudomonas aeruginosa.

Other pulmonary antibiotic therapies have been proposed in the scientific and patent literature. For instance, WO 02/03998 discloses inhalable formulations of macrolide antibiotics, such as erythromycylamine, for delivery by aerosolisation. The concentrated erythromycylamine formulations contain an amount of erythromycylamine effective to treat infections caused by susceptible bacteria. Unit dose devices having a container comprising a formulation of the macrolide antibiotic in a physiologically acceptable carrier are also described. The document further discloses methods for treatment of pulmonary infections by such formulations delivered as an aerosol having mass median aerodynamic diameter predominantly between 1 and 5 micrometers.

In WO 00/35461, a method for the treatment of severe chronic bronchitis (bronchiectasis) using a concentrated aminoglycoside antibiotic formulation is disclosed. The method includes delivering the antibiotic to the lungs endobronchial space including alveoli in an aerosol or dry powder having a mass medium diameter predominately between 1 and 5 microns. The method comprises the administration of the antibiotic at a concentration one to ten thousand times higher than the minimal inhibitory concentration of the target organism. Preferably, the method comprises the endobronchial administration of aerosolized tobramycin to treat pseudomonal infections in severe chronic bronchitis patients.

A wide variety of gram-negative bacteria cause severe pulmonary infections, and many of these bacteria are or become resistant to commonly used or specialty antibiotics including tobramycin, and require treatment with new types of antibiotics. The pulmonary infections caused by gram-negative bacteria are particularly dangerous to patients who have decreased immunoprotective responses, such as cystic fibrosis (CF) and HIV patients, patients with chronic obstructive pulmonary disease (COPD) bronchiectasis or those on mechanical ventilation. Thus, bacterial respiratory infections caused by resistant bacteria remains a major problem, particularly in CF, COPD and HIV patients. For example, chronic pulmonary infection with Pseudomonas aeruginosa in patients with cystic fibrosis is a major cause of their high mortality.

In order to address the continuous need for an effective therapy for treatment of acute and chronic pulmonary bacterial infections caused by gram-negative bacteria and particularly those caused, for example, by Burkholderia cepacia, Stenotrophomonas maltophilia, Alcaligenes xylosoxidans, and multidrug resistant Pseudomonas aeruginosa, WO 02/051356 proposes the local therapy of the respiratory system by delivering a concentrated formulation of the monobactam antibiotic aztreonam as an inhalable aerosol, or as a dry powder formulation. According to the document, about 1 to 250 mg of aztreonam may be dissolved in 1 to 5 ml of saline or another aqueous solution. The formulation is delivered to the lung endobronchial space as an aerosol having mass medium average diameter particles predominantly between 1 and 5 micrometers, using a nebulizer capable of atomizing the aztreonam solution into droplets or particles of the required sizes. Alternatively, for the delivery of a dry inhalable powder, aztreonam is milled or spray-dried to particle sizes of 1 to 5 micrometers.

US 2004/0009126 describes an inhalation system for prevention and treatment of intracellular infection in the lung. The system represents a pharmaceutical formulation comprising colloidal particles such as liposomes and lipid complexes loaded with an anti-infective agent in combination with an inhaler. The anti-infective agent is optionally an antibiotic from the classes of penicillins, tetracyclines, quinolones, cephalosporins, monobactams, aminoglycosides, macrolides etc. The inhalation device may be a dry powder inhaler, a metered-dose inhaler or a nebuliser.

While the document suggests potentially useful active compounds for fighting infections of the lungs, it requires that a formulation is used which comprises the drug substance in form of colloidal particles, in particular liposomes or lipid complexes. Depending on the specific active compound which is selected, one or more disadvantages may be associated with its teachings.

US 2006/0276473 describes aerosol compositions comprising the combination of pentamidine with levofloxacin. In a first aspect, a water-soluble active compound may be difficult to associate efficiently with a lipid-based colloidal drug carrier system. Secondly, pharmaceutical compositions for inhalation use must be sterile for safety reasons, whereas the suggested formulations comprising lipid-based colloidal drug carrier systems are difficult to manufacture in sterile form. Thirdly, a size range of the carrier particles of up to 2 microns is suggested, which poses a considerable difficulty with respect to converting the formulation into an aerosol having a particle size distribution in a range which is suitable for delivery of the aerosol to the lungs. Fourthly, lipid-based colloidal drug carrier systems usually require a large excipient load relative to the content of active ingredient; and if the dose of the active ingredient is relatively high as in the case of many anti-infective compounds (e.g. in comparison to corticosteroids or betamimetics for inhalation), the resulting content of excipients - in particular of amphiphilic lipids - in any aqueous based liquid formulation would result in viscosity which is too high to allow efficient aerosolisation. On the other hand, if such formulation is diluted to lower the viscosity and enable more efficient aerosolisation, the volume of the liquid will increase, which will potentially extend the required inhalation time beyond acceptability.

Thus, there is a need for further pharmaceutical compositions, aerosols and therapeutic kits which are suitable for the prevention, management or treatment of airway infections and which overcome one or more of the disadvantages of presently known therapies. It is therefore an object of the present invention to provide such compositions, aerosols and kits. Further objects will become clear on the basis of the following description and the patent claims.

### SUMMARY OF THE INVENTION

The invention provides a pharmaceutical aerosol for nasal, sinunasal or pulmonary administration comprising a dispersed liquid phase and a continuous gas phase. The dispersed liquid phase consists of aqueous droplets comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts. The droplets of the dispersed phase have a mass median diameter from about 1.5 to about 6 µm, with the droplet size distribution exhibiting a geometrical standard deviation from about 1.2 to about 3.0.

In another aspect, the invention provides a liquid pharmaceutical composition from which such aerosol can be prepared. The liquid composition comprises 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and a volume of not more than about 10 ml and more preferably less than 5 ml of the composition comprises an effective dose of the active compound.

Furthermore, the invention provides a kit comprising a nebuliser and the above described liquid composition, wherein the nebuliser is adapted to aerosolise the liquid composition into an aerosol as described above.

The invention further discloses a method of preparing an aerosol for delivery to a person in need of nasal, sinunasal or pulmonary antibiotic treatment or prophylaxis. The method comprises the step of providing a liquid pharmaceutical composition comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and an effective dose of the active compound in a volume of not more than about 10 ml and more preferably less than 5 ml, and the step of providing a nebuliser capable of aerosolising said liquid pharmaceutical composition at a total output rate of at least 0.1 ml/min, the nebuliser further being adapted to emit an aerosol comprising a dispersed phase having a mass median diameter from about 1.5 to about 6 µm and a geometrical standard deviation from about 1.2 to about 3.0. In a subsequent step, the nebuliser is operated to aerosolise the liquid composition, thus providing a pharmaceutical aerosol which can be inhaled by a patient in need of antibiotic therapy.

The active compound is levofloxacin, including the pharmaceutically acceptable salts, solvates or isomers thereof.

### DETAILED DESCRIPTION OF THE INVENTION

According to a first aspect, the invention provides a pharmaceutical aerosol for nasal, sinunasal or pulmonary administration comprising a dispersed liquid phase and a continuous gas phase, wherein the dispersed liquid phase consists of aqueous droplets comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts. The dispersed phase is further characterised by a mass median diameter from about 1.5 to about 6 µm and a geometrical standard deviation of the droplet size distribution in the range from about 1.2 to about 3.0.

As used herein, an aerosol is a dispersion of a solid or liquid phase in a gas phase. The dispersed phase, also termed the discontinuous phase, is comprised of multiple solid or liquid particles. In general, the particle size of the dispersed phase is typically (considerably) less than about 100 µm. Both basic physical types of aerosols, i.e. solid and liquid dispersions in a gas phase, may be used as pharmaceutical aerosols. Examples of aerosols representing solid particles in a gas phase are those emitted by dry powder inhalers (DPI's). In contrast, pressurised metered-dose inhalers and nebulisers deliver aerosols whose dispersed phase is liquid.

According to the present invention, the aerosol comprises a dispersed liquid phase and a continuous gas phase. Such aerosols are sometimes referred to as "liquid aerosols" or, probably more appropriately, aerosolised liquids. It should be noted that the requirement of a dispersed liquid phase does not exclude the presence of a solid phase. In particular, the dispersed liquid phase may itself represent a dispersion, such as a suspension of solid particles in a liquid.

The continuous gas phase may be selected from any gas or mixture of gases which is pharmaceutically acceptable. For example, the gas phase may simply be air or compressed air, which is most common in inhalation therapy using nebulisers as aerosol generators. Alternatively, other gases and gas mixtures, such as air enriched with oxygen, or mixtures of nitrogen and oxygen may be used. Most preferred is the use of air as continuous gas phase.

An active compound is a natural, biotechnology-derived or synthetic compound or mixture of compounds useful for the diagnosis, prevention, management, or treatment of a disease, condition, or symptom of an animal, in particular a human. Other terms which may be used as synonyms of active compound include, for example, active ingredient, active pharmaceutical ingredient, therapeutic compound, drug substance, drug, and the like.

According to the invention, the active compound is levofloxacin, which is an antibiotic compound selected from the group of quinolone antibiotics. Quinolone antibiotics, also referred to as fluoroquinolones or fluoroquinolone antibiotics, represent a class of broad-spectrum antibiotics which are chemically more or less related to nalidixic acid. Quinolones act by inhibiting the bacterial DNA gyrase or the topoisomerase IV enzyme, or both. Consequently, they inhibit DNA replication and act bacteriocidically.

The aerosol comprises the second generation quinolone levofloxacin.

It should be noted that, in the present description, levofloxacin should be understood so as to include the respective salts, solvates, structural isomers and stereoisomers.

Salts, derivatives, solvates, and isomers are all categories of forms in which an active principle may be used as an active ingredient in a pharmaceutical composition. Salts are neutral compounds composed of ions, i.e. cations and anions. If the active principle can act like an acid, potentially useful salts may be formed with inorganic cations such as sodium, potassium, calcium, magnesium, and ammonium, or with organic cations such as those derived from arginine, glycine, ethylenediamine, and lysine. If the active principle can act like a base, potentially useful salts may be formed with inorganic anions such as chloride, phosphate (mono- or dibasic), sulfate, bromide, iodide, nitrate, acetate, trifluoroacetate, propionate, butyrate, maleate, fumarate, methanesulfonate, ethanesulfonate, 2-hydroxyethyl-sulfonate, n-propylsulfonate isopropylsulfonate, lactate, malate or citrate.

Derivatives are broadly defined as compounds which are chemically and functionally similar to a parent compound. For example, prodrugs may be understood as derivatives of active compounds or active principles which are converted into the respective pharmacologically active species in vivo, such as be chemical or enzymatic hydrolysis or oxidation. Other examples of derivatives are conjugates, analogues, and mimics.

Solvates are compounds formed by solvation, i.e. by the combination of active compounds with molecules of a solvent. An example of a typical solvent is water, and hydrates are well-known examples of pharmaceutically acceptable solvates. As used herein, hydrates include hemihydrates, monohydrates, dihydrates, and any other stoichiometries.

Isomers are molecules with the same chemical formula and often with the same kinds of bonds between atoms, but in which the atoms are arranged differently. Many isomers share similar but not always identical properties. As used herein, isomers include structural isomers and stereoisomers. In structural isomers, the atoms or functional groups are joined together in different ways, whereas in stereoisomers the bond structure is the same, but the geometrical positioning of atoms and functional groups in space differs. Stereoisomers include optical isomers (e.g. enantiomers) where different isomers are mirror-images of each other, and diastereomers and other geometric isomers where functional groups at the end of a chain can be twisted in different ways.

The active compound is levofloxacin, again including the pharmaceutically acceptable salts, solvates and isomers thereof.

The aerosol of the invention is for the pulmonary, nasal or sinunasal delivery. The nose is, anatomically, a protuberance in vertebrates that houses the nostrils, or nares, which admit and expel air for respiration. In humans, as in most other mammals, it also houses the nose hairs, which catch airborne particulate contaminants and prevent them from reaching the lungs. Within and behind the nose are the olfactory mucosa and the paranasal sinuses. Behind the nasal cavity, air next passes through the pharynx, shared with the digestive system, and then into the rest of the respiratory system.

The paranasal sinuses consist of four pairs of air-filled cavities or spaces within the bones of the skull and face. They are divided into subgroups which are named according to the bones they lie under: (1) the maxillary sinuses, also called the antra, which are located under the eyes, in the upper jawbone; (2) the frontal sinuses, which lie above the eyes, in the bone of the forehead; (3) the ethmoid sinuses, positioned between the nose and the eyes, backwards into the skull; and (4) the sphenoid sinuses, which are more or less in the centre of the skull base. While the primary function of the sinuses is not entirely clear, it appears that they decrease the relative weight of the front of the skull, warm and humidify the inhaled air before it reaches the lungs, increase the resonance of the voice, and perhaps provide a buffer against blows to the face.

The nasal cavity and the paranasal sinuses are lined with mucosa. Mucosae, or mucous membranes, are mucus-covered epithelial linings. The mucosae of the nasal cavity and the paranasal sinuses are often affected by bacterial infections with both gram-positive and gram-negative bacteria, and the aerosol of the invention provides improved means to deliver therapeutically useful active agents to these membranes.

In another preferred embodiment, the aerosol is for pulmonary delivery, which is preferably achieved via oral inhalation of the aerosol. As used herein, pulmonary delivery means aerosol delivery to any part or feature of the lungs, including the so-called deep lungs, the peripheral lungs, the alveoli, the bronchi and the bronchioli.

Conditions of the nasal, sinunasal or pulmonary target regions in which the prevention, management, or treatment of a human using the aerosol of the invention is potentially useful include, for example:
- Acute or chronic sinusitis or rhinosinusitis, with or without acute exacerbations, optionally due to Streptococcus pneumoniae, Haemophilus influenza or Moraxella catarrhalis;
- Acute bacterial exacerbations in chronic bronchitis or in chronic obstructive pulmonary disease, optionally due to Staphylococcus aureus, Streptococcus pneumonia, Haemophilus influenza, Haemophilus parainfluenza or Moraxella catarrhalis;
- Nosocomial pneumonia, optionally due to Staphylococcus aureus, Pseudomonas aeruginosa, Serratia marcescens, Escherichia coli, Klebsiella pneumoniae, Haemophilus influenza or Streptococcus pneumoniae; and
- Community acquired pneumonia, optionally due to Staphylococcus aureus, Streptococcus pneumoniae, Haemophilus influenza, Haemophilus parainfluenza, Klebsiella pneumoniae, Moraxella catarrhalis, Chlamydia pneumoniae, Legionella pneumophila, or Mycoplasma pneumoniae.

In particular in immuno-compromised patients, such as persons suffering from HIV infection, sensitive patients, persons receiving immunosuppressive drugs, or cystic fibrosis patients, the aerosol of the invention may be useful not only for the treatment or management of an existing bacterial infection, but for the prevention or prophylaxis of such infection.

The aerosol is further characterised in that its dispersed liquid phase exhibits a mass median diameter in the range from about 1.5 to about 6 µm, with a geometric standard deviation of the droplet size in the range from about 1.2 to about 3.0. The mass median diameter (MMD), as used herein, is the mass median diameter of the dispersed liquid phase as measured by laser diffraction. Various appropriate analytical apparatuses to determine the mass median diameter are known and commercially available, such as the Malvern MasterSizer X or Malvern SprayTec. The geometric distribution including the geometric standard deviation (GSD) of the aerosolised liquid particles or droplets may be determined simultaneously with the mass median diameter. The geometric standard deviation describes how spread out are a set of numbers whose preferred average is the geometric mean.

In one of the preferred embodiments, the aerosol is for pulmonary delivery, and exhibits a MMD in the range from about 2.0 to about 4.5 µm and a GSD in the range from about 1.2 to about 1.8. More preferably, the aerosol of the invention, if adapted for pulmonary delivery, has a MMD in the range from about 2.5 to about 4.0 and a GSD in the range from about 1.4 to about 1.6. It has been found that each of these sets of combinations is particularly useful to achieve a high local drug concentration in the lungs, including the bronchi and bronchioli, relative to the amount of drug which is aerosolised.

In another preferred embodiment, the aerosol is for sinunasal delivery. According to this embodiment, the MMD is preferably in the range from about 2.5 to 4.5 µm, in others from about 3 to about 4 µm, or from about 2.8 to about 3.5 µm, respectively. For this embodiment, it is also preferred that the GSD is in the range from about 2.3 to about 3.0 or, more preferably, at least about 2.4, or at least about 2.5, respectively. Additionally, if the aerosol is for sinus or sinunasal delivery, it is preferred that it pulsates (or vibrates) with a selected frequency. As used herein, the pulsation of an aerosol is understood as a periodic change of pressure. Preferably, the pulsation is regular, i.e. the time interval between pressure peaks is approximately constant. The amplitude of pressure pulsation may also be relatively constant, at least with regard to the generation and emission of the pulsating aerosol from the aerosol generator. Preferably, the pressure of the aerosol pulsates with a frequency in the range from about 10 Hz to about 90 Hz. According to some of the presently preferred embodiments, the pressure may also pulsate at a frequency in the range from about 10 to about 60 Hz, or from 10 to about 55 Hz, or from about 30 to about 60 Hz. In a further embodiment, the aerosol vibrates at a frequency of about 30 to about 55 Hz, such as from about 40 to about 50 Hz, for example about 44 Hz.

It has been found that a vibrating aerosol enters the paranasal sinuses after nasal inhalation to a much larger extent than a conventional aerosol having a substantially constant pressure, provided that the appropriate particles sizes are selected as outlined above. Larger particle sizes will lead to little sinus deposition, but to a large deposition on the nasal mucosa, whereas very small particle sizes allow the aerosol droplets to enter the sinuses following the pressure gradient of a pressure pulse, but also their exit from the sinuses without them being deposited therein.

In another preferred embodiment, the aerosol is emitted from an aerosol generator at a rate of at least about 0.1 ml/min. In another embodiment, the (total) output rate, which describes the rate at which the aerosol is emitted from the aerosol generator, is at least about 0.150 ml/min, or at least about 150 mg/min for those liquid aerosols whose density is, for practical purposes, close to 1, i.e. within the range from about 0.95 to about 1.05. In further embodiments, the output rate is from about 200 to about 800 mg/min, or from about 250 to about 750 mg/min, respectively.

Appropriate aerosol generators, in particular nebulisers, which are suitable for generating the aerosol described herein at a rate of 0.1 ml/min or more will be discussed in further detail herein-below.

The aerosol may optionally comprise at least one further active compound which may, for example, be selected from non-quinolone antibiotics, efflux pump inhibitors, compounds acting against bacterial biofilms, antifungals, antivirals, immunomodulators, lung surfactant, beta agonists, anticholinergics, mucolytics, heparinoids, antiinflammatory and antiallergic drugs.

In another aspect, the present invention is directed to a liquid pharmaceutical composition for preparing an aerosol as described above. The liquid composition comprises a unit dose of the active compound in a volume of not more than about 10 ml.

As defined herein, a liquid pharmaceutical composition is a liquid material which comprises at least one active compound and at least one pharmaceutically acceptable, pharmacologically substantially inert excipient. It should be noted that the term "liquid composition" does not necessarily mean that no solid material is present. For example, a liquid suspension representing a dispersion of solid particles in a continuous liquid phase is also covered by the expression.

Preferably, the liquid composition from which the aerosol is prepared is an aqueous composition, which means that water represents the predominant liquid constituent of the composition. Solvents and co-solvents, other than water, should be avoided if possible. In another embodiment, the composition comprises at least 80 wt.-% of water. In yet another embodiment, at least about 90 wt.-% of the liquid constituents of the composition is water.

If the incorporation of a solvent cannot be avoided, the excipient should be selected carefully and in consideration of its physiological acceptability. For example, if the composition is designated for the treatment of a life-threatening disease, the use of some limited amount of ethanol, glycerol, propylene glycol or polyethylene glycol as a non-aqueous solvent may be acceptable. According to the presently more preferred embodiments, however, the composition is substantially free of these solvents, and in particular of glycerol, propylene glycol or polyethylene glycol.

As mentioned, the volume of the liquid composition which comprises an effective dose, or a unit dose, of the active ingredient is not more than about 10 ml. More preferably, the volume is about 5 ml or less. This is to ensure patient convenience and compliance: Volumes of more than 5 ml are associated with relatively long inhalation times which are not well-accepted with many patients, perhaps with the exception of patients suffering from particularly severe acute conditions. According to a further embodiment, the volume of the liquid composition comprising an effective dose of the active compound is about 4 ml or less, and in a yet further embodiment it is about 2.5 ml or less. In addition the inhalation treatment time needed to deposit a therapeutic effective dose either to the sinusoidal cavities or into the lung is less than 15 min and more preferably less than 10 min and ideally less than 5 minutes.

The dynamic viscosity of the liquid composition has an influence on the particle size distribution of the aerosol formed by nebulisation and on the efficiency of nebulisation. It should preferably be adjusted to a range of about 0.8 to about 3 mPas. According to another embodiment, the dynamic viscosity is in the range of about 1.0 to about 2.5 mPas.

To obtain an aerosol which is highly suitable for the preferred uses described herein, the surface tension of the liquid composition of the invention should preferably be adjusted to the range of about 25 to 80 mN/m, and preferably to the range of about 30 to 75 mN/m. In this context, it is to be taken into consideration that, in the lowest part of this range, a particularly good spreadability of the preparation on the mucous membranes may be expected, but that the quality of the aerosol and the efficiency of the nebulisation could be adversely affected.

On the other hand, if the incorporation of a surfactant appears necessary, e.g. for stabilising or solubilising a poorly soluble active agent, it can hardly be avoided that the surface tension is reduced fairly markedly below that of water or physiological buffer solution. Thus, a compromise may have to be found in each case depending on the active compound and the intended application.

In order to be well-tolerated, an aerosol should, as far as possible, have a physiologic tonicity or osmolality. Thus, it may be desirable to incorporate an osmotically active excipient to control the osmolality of the aerosol. The content of this excipient (or excipients, if a combination of substances is used) should be selected to yield an osmolality of the aerosol which does not deviate too much from that of physiological fluids, i.e., from about 290 mOsmol/kg. However, in individual cases, a compromise has again to be found between the physical-chemical or pharmaceutical needs on one hand and the physiological requirements on the other hand. Furthermore, it is believed that sinunasal aerosol delivery is not as problematic in terms of osmolality as, for example, deep lung delivery of aerosols. In general, an osmolality in the range of up to 800 mOsmol/kg may be acceptable. In particular, an osmolality in the range of about 200 up to about 600 mOsmol/kg is preferred. In further embodiments, the osmolality is even closer to the physiological value, i.e. from about 220 to about 400 mOsmol/kg.

To achieve a particularly high effectiveness against bacteria in the target regions, it may be useful to enhance the local retention of the composition after deposition of the aerosol. For example, a prolonged residence time of the composition deposited in the lungs may lead to a higher continuous exposure of the pathogens to the antibiotic agent. At the same time, it may reduce the required frequency of administration and thus enhance patient convenience and compliance.

Various formulation strategies may be pursued to achieve a prolonged retention. Firstly, it possible to incorporate a highly water soluble active compound in a less soluble solid form, such as in the form of a poorly soluble salt. This will require that the compound is present in the aerosol in undissolved form, such as in form of a micro- or nanosuspension. Upon deposition of the aerosol droplets, the liquid phase of the composition combines with physiological fluid, e.g. mucus, and allow the drug substance to dissolve.

In one of the preferred embodiments, the active compound is formulated with a polymeric excipient to effect slow release and prolonged local retention. Potentially suitable polymers include, in particular, pharmaceutically acceptable water-soluble or water-dispersible polymers, such as methylcellulose, hydroxymethylcellulose, hydroxyethylcellulose, hydroxypropylcellulose, hydroxybutylcellulose, hydroxyethyl methylcellulose, hydroxypropyl methylcellulose, carboxymethylcellulose, carboxymethyl ethylcellulose, alginate, carrageen, galactomannan, tragacanth, agar, acacia, guar gum, xanthan gum, pectin, carboxymethyl amylopectin, chitosan, dextran, polyacrylate, polymethacrylate, methacrylate copolymer, polyvinyl alcohol, polyvinylpyrrolidone, polyvinylpyrrolidone vinyl acetate copolymer, and mixtures of any of these.

One of the presently preferred polymeric excipients is chitosan. Chitosan is a linear polysaccharide composed of randomly distributed β-(1-4)-linked D-glucosamine and N-acetyl-D-glucosamine. Chitosan is produced by deacetylating chitin, which is the structural element in the exoskeleton of crustaceans and other animals. The degree of deacetylation in useful chitosans according to the invention is in the range 60-100 %, and more preferably at least about 75 %. The amino group in chitosan has a pKa value of about 6.5, so that chitosan is usually positively charged and soluble in neutral or acidic aqueous media. Chitosan has also bioadhesive properties as it interacts with negatively charged surfaces such as mucosal membranes, which may further contribute to its usefulness in the composition of the invention.

If chitosan is selected as an excipient in the liquid composition, its content should preferably be low enough to not exceed a dynamic viscosity of about 3 mPas in order to allow efficient aerosolisation. As the effect of the excipient on the viscosity will depend on the specific grade of the chitosan in terms of molecular weight and degree of deacetylation, but also on the pH of the composition and on other excipients which may be present. For most grades of chitosan, it is preferred that the content in the composition is not more than about 0.5 wt.-%. In another embodiment, the chitosan content is in the range from about 0.01 to about 0.25 wt.-%. In yet another embodiment, it is in the range from about 0.025 to about 0.1 wt.-%.

A further water soluble polymer which is among the preferred excipients in the composition is selected from the group of water soluble cellulose ethers, such as methylcellulose, hydroxymethylcellulose, hydroxyethylcellulose, hydroxypropylcellulose, hydroxybutylcellulose, hydroxyethyl methylcellulose, hydroxypropyl methylcellulose, carboxymethylcellulose, and carboxymethyl ethylcellulose. Particularly preferred is hydroxypropyl methylcellulose. The cellulose ether may be the only polymeric excipient in the composition, or it may be incorporated in combination with another polymer such as chitosan.

If a cellulose ether such as hydroxypropyl methylcellulose is selected as one of the excipients, the same guidance applies as has been given for selecting the content of chitosan: Care should be taken that the resulting dynamic viscosity does preferably not exceed a value of roughly 3 mPas, as aerosolisation will be more difficult otherwise. Again, the exact grade of the polymer and the presence of other excipients should be taken into account when calculating the content of polymer. As a general rule for most grades of hydroxypropyl methylcellulose, it is preferred that the content in the liquid composition is not more than about 1 wt.-%. In another embodiment, the content is selected in the range from about 0.05 to about 0.5 wt.-%.

It has been found that the polymeric excipient(s) as described above have an effect on the release of the active compound from the formulation, or on the local residence time of the composition after deposition onto the target tissue, or both. In this way, they also affect the local bioavailability of the active compound and the exposure of the microorganisms.

Potentially, other excipients than polymers may also have such beneficial effects. For example, complexing agents may in a similar fashion prolong the release of the active compound as polymeric excipients. The same is true for certain amphiphilic compounds, in particular amphiphilic lipids which may form various types of colloidal structures which interact with the active ingredient and improve its residence time in the target region.

Examples of potentially suitable complexing agents include cyclodextrins. Cyclodextrins (CDs) are cyclic oligosaccharides composed of (α-1,4)-linked α-D-glucopyranose units. They comprise a relatively hydrophobic central cavity and a hydrophilic external region. Because the monomeric units cannot rotate freely at the α-1,4-linkages, the shape of the molecules is more conical than cylindrical, with the primary hydroxyl groups located at the smaller part and the secondary hydroxyl groups at the larger part of the conus.

The most common cyclodextrins are α-, β-, and γ-cyclodextrins with 6, 7, and 8 glucopyranose units, respectively. The diameters of the cavities are approximately 4.7 to 5.3 Å for α-cyclodextrins, 6.0 to 6.5 for β-cyclodextrins, and 7.5 to 8.3 for γ-cyclodextrins. The non-derivatised cyclodextrins exhibit aqueous solubilities of about 145 mg/ml (α-cyclodextrin), 18.5 mg/ml (β-cyclodextrin), and 232 mg/ml (γ-cyclodextrin) at 25 °C.

Cyclodextrins are known for their capability of forming inclusion complexes with smaller molecules. If the host molecules themselves are poorly water-soluble, they may become solubilised in the form of such cyclodextrin inclusion complexes. Several pharmaceutical agents have been successfully formulated into marketed drug products which incorporate cyclodextrins as solubility-enhancing agents.

Examples of potentially useful cyclodextrins include the non-derivatised cyclodextrins, but also derivatives whose hydroxyl groups are alkylated or hydroxyalkylated, esterified, or etherified, such as 2-hydroxypropyl-β-cyclodextrin, 2-hydroxypropyl-γ-cyclodextrin, sulfobutyl-β-cyclodextrin, sulfobutyl-γ-cyclodextrin, maltosyl-P-cyclodextrin, and methyl-β-cyclodextrin. Particularly preferred at present are 2-hydroxypropyl-p-cyclodextrin, and sulfobutyl-β-cyclodextrin, α-cyclodextrin, β-cyclodextrin, and γ-cyclodextrin.

Other potentially suitable complexing agents include di- or multivalent metal salts, in particular calcium-, magnesium-, and aluminium salts, and chelating agents such as ethylenediaminetetraacetic acid including its salts and derivatives. Furthermore, di- or multivalent metal salts may also function as stabilisers of complexes formed by active compounds and polymers, such as chitosan.

Examples of potentially suitable amphiphilic excipients include, in particular, phospholipids. Phospholipids can be defined as amphiphilic lipids which contain phosphorus. Also known as phosphatides, they play an important role in nature, in particular, as double layer-forming constituents of biological membranes. Phospholipids which are chemically derived from phosphatidic acid occur widely and are also commonly used for pharmaceutical purposes. This acid is a usually (doubly) acylated glycerol-3-phosphate in which the fatty acid residues may be of different length. The derivatives of phosphatidic acid include, for example, the phosphocholines or phosphatidylcholines, in which the phosphate group is additionally esterified with choline, furthermore phosphatidyl ethanolamines, phosphatidyl inositols etc. Lecithins are natural mixtures of various phospholipids which usually have a high proportion of phosphatidyl cholines. Depending on the source of a particular lecithin and its method of extraction and/or enrichment, these mixtures may also comprise significant amounts of sterols, fatty acids, triglycerides and other substances.

Suitable phospholipids are also those which are suitable for administration by inhalation on account of their physiological properties. These comprise, in particular, phospholipid mixtures which are extracted in the form of lecithin from natural sources such as soy beans or chickens egg yoke, preferably in hydrogenated form and/or freed from lysolecithins, as well as purified, enriched or partially synthetically prepared phospholipids, preferably with saturated fatty acid esters. Particularly preferred are purified, enriched or partially synthetically prepared medium-to long-chain zwitterionic phospholipids which are mainly free from unsaturation in the acyl chains and free from lysolecithins and peroxides. Of the phospholipid mixtures, lecithin is particularly preferred. Examples for enriched or pure compounds are dimyristoyl phosphatidyl choline (DMPC), distearoyl phosphatidyl choline (DSPC) and dipalmitoyl phosphatidyl choline (DPPC).

Optionally, the liquid composition may comprise further pharmaceutically acceptable excipients, such as osmotic agents, in particular inorganic salts; excipients for adjusting or buffering the pH, such as organic or inorganic salts, acids, and bases; bulking agents and lyophilisation aids, such as sucrose, lactose, mannitol, sorbitol, xylitol, and other sugar alcohols; stabilisers and antioxidants, such as vitamin E or vitamin E derivatives, Lycopene and its derivatives, ascorbic acid, sulphites, hydrogen sulphites, gallic acid esters, butyl hydroxyanisole, and butyl hydroxytoluene; ionic and nonionic surfactants, including phospholipids, such as those surfactants disclosed above; furthermore taste-masking agents, disintegrants, colouring agents, sweeteners, and flavours.

In one of the preferred embodiments, one or more osmotic agents such as sodium chloride are incorporated in the composition to adjust the osmolality to a value in the preferred range as outlined herein-above.

In another embodiment, the composition comprises at least one excipient to adjust the pH. In order to provide a well tolerated aerosol, the preparation according to the invention should be adjusted to a euhydric pH value. The term "euhydric" already implies that there may be a difference between pharmaceutical and physiological requirements so that a compromise has to be found which, for example, guarantees that the preparation is, from an economical point of view, just sufficiently stable during storage but, on the other hand, largely well tolerated. Preferably, the pH value lies in the slightly acidic to neutral region, i.e., between pH values of about 4 to 8. It is to be noted that deviations towards a weakly acidic environment can be tolerated better than shifts of the pH value into the alkaline region. A pH value in the range of about 4.5 to about 7.5 is particularly preferred.

For adjusting and, optionally, buffering pH value, physiologically acceptable acids, bases, salts, and combinations of these may be used. Suitable excipients for lowering the pH value or as acidic components of a buffer system are strong mineral acids, in particular, sulfuric acid and hydrochloric acid. Moreover, inorganic and organic acids of medium strength as well as acidic salts may be used, for example, phosphoric acid, citric acid, tartaric acid, succinic acid, fumaric acid, methionine, acidic hydrogen phosphates with sodium or potassium, lactic acid, glucuronic acid etc. However, sulphuric acid and hydrochloric acid are most preferred. Suitable for raising the pH value or as basic component for buffer system are, in particular, mineral bases such as sodium hydroxide or other alkali and alkaline earth hydroxides and oxides such as, in particular, magnesium hydroxide and calcium hydroxide, ammonium hydroxide and basic ammonium salts such as ammonium acetate, as well as basic amino acids such as lysine, carbonates such as sodium or magnesium carbonate, sodium hydrogen carbonate, citrates such as sodium citrate etc.

In one of the embodiments, the composition of the invention contains a buffer system consisting of two components, and one of the particularly preferred buffer systems contains citric acid and sodium citrate. Nevertheless, other buffering systems may also be suitable.

Not primarily for physiological, but for pharmaceutical reasons the chemical stabilisation of the composition by further additives may be indicated. This depends mainly on the kind of the active agent contained therein. The most common degradation reactions of chemically defined active agents in aqueous preparations comprise, in particular, hydrolysis reactions, which may be limited, primarily, by optimal pH adjustment, as well as oxidation reactions. Examples for active agents which may be subject to oxidative attack are those agents that have olefinic, aldehyde, primary or secondary hydroxyl, ether, thioether, endiol, keto or amino groups. Therefore, in the case of such oxidation-sensitive active agents, the addition of an antioxidant, optionally in combination with a synergist, may be advisable or necessary.

Antioxidants are natural or synthetic substances which prevent or interrupt the oxidation of the active agents. These are primarily adjuvants which are oxidizable themselves or act as reducing agents, such as, for example, tocopherol acetate, lycopene, reduced glutathione, catalase, peroxide dismutase. Synergistic substances are, for example, those which do not directly act as reactance in oxidation processes, but which counteract in oxidation by an indirect mechanism such as the complexation of metal ions which act catalytically in the oxidation, which is the case, for example, for EDTA derivatives (EDTA: ethylenediamine tetraacetic acid). Further suitable antioxidants are ascorbic acid, sodium ascorbate and other salts and esters of ascorbic acid (for example, ascorbyl palmitate), fumaric acid and its salts, malic acid and its salts, butyl hydroxy anisole, propyl gallate, as well as sulphites such as sodium metabisulfite. Apart from EDTA and its salts, citric acid and citrates, malic acid and its salts and maltol (3-hydroxy-2-methyl-4H-pyran-4-one) may also act as chelating agents.

In one of the embodiments, the composition contains at least one antioxidant. In a further embodiment, it contains both an antioxidant and a chelating agent. The combination of a vitamin E derivative, in particular, vitamin E acetate, with an EDTA derivative, in particular, EDTA disodium salt, is particularly preferred. In the case of certain active agents, this combination has proven to be particularly advantageous for obtaining high chemical stability of the composition.

In another embodiment, the composition comprises an excipient which affects the taste. A distinctly bad taste is extremely unpleasant and irritating in inhalation and it can result in non-compliance, and thus, therapy failure. The bad taste is perceived by the patient through the part of the aerosol which precipitates in the oral and pharyngeal region during inhalation. Even if it can be achieved by optimising the particle size of the aerosol that a low a fraction of the preparation precipitates there (this fraction is also lost for therapy, unless the oral pharyngeal or nasal mucosa are the tissue to be treated) it is presently hardly possible to reduce this fraction to such an extent that the bad taste of an active agent would no longer be perceived. Therefore, the improvement of the taste of the composition or the masking of the poor taste of a selected active compound is recommendable.

To improve the taste, one or more potentially useful excipients from the group of sugars, sugar alcohols, salts, flavours, complexing agents (e.g. cyclodextrins), polymers, sweeteners (e.g. saccharin sodium, aspartate, arginine etc.) and surfactants may be incorporated.

As mentioned above, the active compound is levofloxacin including the pharmaceutically acceptable salts, solvates and isomers thereof.

The content of active ingredient should be selected to ensure that the minimum inhibitory concentration (MIC) at the target site is exceeded. More preferably, it is selected to exceed the MIC by a factor of at least 2, or of at least 3, respectively. Of course, not always is the exact MIC for a particular pathogen known, and it may be useful to provide the composition having an active compound concentration which is effective against a large number of bacterial strains.

In one of the preferred embodiments, the composition comprises from about 10 to about 500 mg of levofloxacin or the equivalent amount of a salt or solvate or isomer thereof, per unit dose, which is the portion of the composition designated for a single administration. More preferably, the dose of levofloxacin is in the range from about 25 to about 250 mg, such as from about 50 to about 200 mg. The selection of the exact dose to be incorporated should also take the aerosolisation efficiency of the aerosol generator into account, as will be discussed more in detail below.

The liquid composition of the invention exhibits a levofloxacin concentration in the range from 20 to 200 mg/ml. More preferably, the concentration is from about 20 to about 150 mg/ml, or from about 25 to about 100 mg/ml, respectively. It has been found that such concentration is very compatible with the preferred types of aerosol generators, allows highly efficient aerosolisation, and is still very tolerable.

Optionally, the composition comprises a further active compound, such as another antibiotic with a complementary antibacterial spectrum or a synergist, such as a pump efflux or biofilm forming inhibitor or a bioadhesive or compound which facilitates the influx of the antibiotic through the bacteria cell membrane into the bacteria.

In another embodiment, the aerosol of the invention comprises an active ingredient selected from the chemically less stable quinolone antibiotics. In this case, a liquid formulation for aerosolisation may not have a sufficiently long shelf life to serve as a suitable market formulation, and it may be advantageous to provide a solid composition instead. Typically, a solid composition of a chemically unstable active compound has the potential for a longer shelf life.

The dry solid composition preferably comprises the active compound and at least one excipient. In general, the same excipients may be selected as described above. Depending on the manufacturing method of the solid composition, one or more additional excipients may be useful. For example, if the composition is prepared by freeze drying (lyophilisation), which is one of the particularly preferred methods of preparing such solid composition according to the invention, it may be useful to incorporate at least one bulking agent and/or lyophilisation aid, such as a sugar or a sugar alcohol, in particular sucrose, fructose, glucose, mannitol, sorbitol, trehalose, isomalt, or xylitol.

The solid composition is further characterised in that the portion of it which comprises an effective amount of the active compound, or a unit dose, is dissolvable or dispersible in an aqueous solvent having a volume of not more than about 10 ml. In another embodiment, it is dissolvable or dispersible in an aqueous liquid volume of not more than about 5 ml, or not more than about 4 or even 2 ml, respectively. In addition nebulisation or inhalation takes less than 15 min and more preferably less than 5 minutes.

As defined herein, dissolvable means that the solid composition and the aqueous liquid solvent can be combined to form a solution or colloidal solution, whereas the term "dispersible" should be interpreted to also include the formation of liquid dispersions, in particular emulsions and microsuspensions.

Aqueous means that the major liquid constituent of the solvent is water. Solvents and co-solvents, other than water, should be avoided if possible. In another embodiment, the aqueous liquid solvent comprises at least 80 wt.-% of water. In yet another embodiment, at least about 90 wt.-% of the liquid constituents of the solvent is water.

If the incorporation of a solvent cannot be avoided, the excipient should be selected carefully and in consideration of its physiological acceptability. For example, if the aerosol is designated for the treatment of a life-threatening disease, the use of some limited amount of ethanol, glycerol, propylene glycol or polyethylene glycol as a non-aqueous solvent may be acceptable. According to the presently more preferred embodiments, however, the aqueous solvent to dissolve or disperse the solid composition is substantially free of these solvents, and in particular of glycerol, propylene glycol or polyethylene glycol.

The solid composition for reconstitution may be part of a pharmaceutical kit. Such kit preferably comprises the solid composition in sterile form. As used herein, the term "sterility" is to be defined according to the usual pharmaceutical meaning. It is understood as the absence of germs which are capable of reproduction. Sterility is determined with suitable tests which are defined in the relevant pharmacopoeias. According to current scientific standards, a sterility assurance level of 10⁻⁶ is generally regarded as acceptable for sterile preparations, i.e., one unit in a million might be contaminated.

In practice, however, contamination rates may be higher. For example, it is generally assumed that the contamination rate for aseptically manufactured preparations might amount to about 10⁻³. Since, on one hand, the extent of sterility tests for quality control of lots according to the pharmacopeias is limited and, on the other hand, contaminations may be caused as artefacts while carrying out the test itself, it is difficult to demand sterility in an absolute sense or to test a particular product for it. Therefore, the sterility of the composition should be understood herein such that the composition meets the requirements with respect to sterility of the relevant pharmacopeia. The same applies to the liquid formulations which are ready to use.

As mentioned above, the solid composition may be prepared by providing a liquid composition which is similar to the liquid composition to be aerosolised, and subsequently drying it, such as by lyophilisation. Similar means that the liquid composition from which the solid composition is prepared by drying may not comprise all solid ingredients of the ready-to-use liquid composition, for example in the case that the liquid carrier for reconstitution is designed to comprise one or more of the excipients. Also, it is not necessary that the concentrations of the ingredients are identical for these two liquid compositions. Alternatively, the solid composition for reconstitution may be prepared by providing the active ingredient and, optionally, at least one excipient, in powder form and subsequently mixing these to form a powder mixture.

In another aspect, the present invention is directed to a pharmaceutical kit for the preparation and delivery of an aerosol for nasal, sinunasal or pulmonary administration, comprising a dispersed liquid phase and a continuous gas phase, wherein the dispersed liquid phase consists of aqueous droplets comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, has a mass median diameter from about 1.5 to about 6 µm, and droplet size distribution exhibiting a geometrical standard deviation from about 1.2 to about 3.0. The kit is further characterised in that it comprises an aerosol generator selected from the group of nebulisers and an aqueous liquid composition, said composition comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and comprising an effective dose of the active compound within a volume of not more than about 10 ml.

Nebulisers are devices capable of aerosolising liquids. Preferably, the nebuliser of the kit of this embodiment of the invention is selected from jet, ultrasonic or piezoelectric, jet collision, electrohydrodynamic, capillary force, perforated membrane nebulisers or perforated vibrating membrane nebulisers. (M.Knoch and M. Keller: The customised electronic nebuliser: a new category of liquid aerosol drug delivery systems. Expert Opin. Drug Deliv. 2005, 2 (2), 377-390). Particularly preferred are piezoelectric, electro-hydrodynamic and/or perforated membrane-type nebulisers, for example, Mystic™, eFlow™, AeroNeb™, AeroNeb Pro™, Aero Dose™. These types of nebulisers are particularly useful if the aerosol is to be delivered to the bronchi and/or lungs.

If the aerosol is to be delivered to the nasal or sinunasal cavities or regions, it is preferred that the nebuliser is selected from the group of jet nebulisers capable of emitting a pulsating (or vibrating) aerosol. Such modified jet nebuliserss with which the sinuses may be reached much better than previously have recently become available. These nebulisers have a nose piece for directing the aerosol flow into the nose. If only one nostril is used for inhalation of the aerosol, the other nostril must be closed by a suitable restrictor. Furthermore, these nebulisers are characterized in that they release an aerosol with pulsating pressure. The pulsating pressure waves achieve a more intensive ventilation of the sinuses so that a concomitantly inhaled aerosol can spread better into these cavities. Examples for such nebulization devices are disclosed in DE 102 39 321 B3.

Preferably, the nebuliser should be selected or adapted to be capable of aerosolising a unit dose, i.e. a volume of the liquid composition comprising the effective amount of active compound which is designated to be administered during a single administration, at a rate of at least about 0.1 ml/min or, assuming that the relative density of the composition will normally be around 1, at a rate of at least about 100 mg/min. More preferably, the nebuliser is capable of an output rate of at least about 0.15 ml/min or 150 mg/min, respectively. In further embodiments, the output rates of the nebuliser are at least about 0.2, 0.3, 0.4, or 0.5 ml/min, respectively.

Furthermore, the output rate of the nebuliser should be selected to achieve a short nebulisation time of the liquid composition. Obviously, the nebulisation time will depend on the volume of the composition which is to be aerosolised and on the output rate. Preferably, the nebuliser should be selected or adapted to be capable of aerosolising a volume of the liquid composition comprising an effective dose of the active compound within not more than about 20 minutes. More preferably, the nebulisation time for a unit dose is not more than about 10 minutes. In further embodiment, the nebuliser is selected or adapted to enable a nebulisation time per dose unit of not more than about 8 minutes, or not more than about 5 minutes. Presently most preferred is a nebulisation time in the range from about 1 to about 8 minutes.

The nebuliser should also preferably be selected to be capable of emitting an aerosol wherein a substantial fraction of the dispersed phase has a droplet size of not more than 5 µm. This fraction is often referred to as the respirable fraction, as these droplets - in contrast to larger droplets - have a high chance of being deposited in the lungs, instead of in the trachea and the pharynx. In one of the embodiments, the nebuliser is selected to emit an aerosol having a respirable fraction of at least about 50 wt.-% of the aerosol. More preferably, the respirable fraction is at least about 70 wt.-%. Particularly preferred are nebulisers which are adapted to generate an aerosol from the liquid composition described above which has a respirable fraction of about 80 wt.-% or more, or even about 90 wt.-% or more, respectively.

According to a further preference, the nebuliser is adapted to deliver the major fraction of the loaded dose of liquid composition as aerosol, such as at least about 50 wt.-% of the loaded dose. More preferably, at least 60 wt.-% of the dose filled into the nebuliser are actually emitted from the device, which is best achieved by using a modern, optionally customised electronic nebuliser based on the vibrating perforated membrane design. According to another embodiment, at least about 70 wt.-% of the loaded dose is aerosolised, or even at least about 75 wt.-% or 95 wt.-%, respectively.

Also provided by the present invention is a method of preparing an aerosol which comprises the steps of providing a liquid pharmaceutical composition comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and comprising an effective dose of the active compound in a volume of not more than about 10 ml and more preferably less than 5 ml; providing a nebuliser capable of aerosolising said liquid pharmaceutical composition at a total output rate of at least 0.1 ml/min, the nebuliser further being adapted to emit an aerosol comprising a dispersed phase having a mass median diameter from about 1.5 to about 6 µm and a geometrical standard deviation from about 1.2 to about 3; and operating the nebuliser to aerosolise the liquid composition. Preferably, the nebulisation of the composition is conducted within a period of not more than about 15 minutes and more preferably less than 5 minutes.

The composition of the invention, whether liquid or initially solid, or the kit which comprises the composition, is useful for the prophylaxis, management or treatment of:
- Acute or chronic sinusitis or rhinosinusitis, with or without acute exacerbations, optionally due to Streptococcus pneumoniae, Haemophilus influenza or Moraxella catarrhalis;
- Acute bacterial exacerbations in chronic bronchitis or in chronic obstructive pulmonary disease, optionally due to Staphylococcus aureus, Streptococcus pneumoniae, Haemophilus influenza, Haemophilus parainfluenza or Moraxella catarrhalis;
- Nosocomial pneumonia, optionally due to Staphylococcus aureus, Pseudomonas aeruginosa, Serratia marcescens, Escherichia coli, Klebsiella pneumoniae, Haemophilus influenza or Streptococcus pneumoniae; and
- Community acquired pneumonia (CAP), hospital acquired pneumonia (HAP) or ventialator associatetd pneumonia (VAP) optionally due to Staphylococcus aureus, Streptococcus pneumoniae, Haemophilus influenza, Haemophilus parainfluenza, Klebsiella pneumoniae, Moraxella catarrhalis, Chlamydia pneumoniae, Legionella pneumophila, or Mycoplasma pneumoniae.

In particular in immuno-compromised patients, such as persons suffering from HIV infection, sensitive patients, persons receiving immunosuppressive drugs, or cystic fibrosis patients, the composition or kit of the invention may be useful not only for the treatment or management of an existing bacterial infection, but for the prevention or prophylaxis of such infection.

Preferably, the composition is administered using a regimen of repeated administration over a course of at least about one week. Optionally, the duration of the regimen is about 10 days or about 2 weeks. In further embodiments, the duration is from about 3 weeks to about 3 months. Furthermore, the regimen preferably comprises once or twice daily inhalation; most preferred is once daily administration over the course of therapy.

The following examples serve to illustrate the invention.

### EXAMPLES

### Example 1:

Levofloxacin hemihydrate (2.5 g), chitosan (0.06 g), hydroxypropyl methylcellulose (0.20 g), magnesium sulfate hexahydrate (0.13 g), and sodium chloride (0.71 g) were dissolved in water for injection to a total weight of 100.0 g. The solution was sterile filtered, using a 0.22 µm filter and 1.5 ml each were filled under aseptic conditions in a laminar air flow hood into 2 ml preformed sterile blow fill seal vials, which were heat sealed afterwards.

The liquid composition had a dynamic viscosity of 2.1 mPas. The surface tension was 47.8 mN/m. The pH at 22 °C was 6.73, and the osmolality was 332 mOsmol/kg.

### Example 2:

Levofloxacin hemihydrate (3.5 g), 2-HP-β-Cyclodextrin (5 g), magnesium sulfate hexahydrate (0.15 g), and sodium chloride (0.25 g) were dissolved in water for injection to a total weight of 100 g. The resulting solution was sterile filtered, using a 0.22 µm filter and 10 ml were filled under aseptic conditions into 15 ml glass vials. The solution had an osmolality of 292 mOsmol/kg.

### Example 3:

Levofloxacin hemihydrate (4 g), Tobramycin (6 g), 2-HP-β-Cyclodextrin (10 g), magnesium sulfate hexahydrate (0.15 g), and sodium chloride (0.25 g) were dissolved in water for injection to a total weight of 100 g. 1 ml each were filled under aseptic conditions in a laminar air flow hood into 2 ml preformed blow fill seal vials, which were heat sealed afterwards.

### Example 4:

Levofloxacin hemihydrate (5 g), Heparin Sodium (1 g), Gamma-Cyclodextrin (15.0 g), magnesium sulfate hexahydrate (0.15 g), lecithin (0.25g) and sodium chloride (0.25 g) were dissolved in water for injection to a total weight of 100.0 g. Both solutions were sterile filtered, using a 0.22 µm filter and filled into 5 ml sterile amber glass vials. Both solutions had an osmolality value in the range of 290-350 mOsmol/kg.

### Example 5: (for comparison and/or reference only)

Kollidon SR (0.3 g) was dissolved in ethanol. Levofloxacin hemihydrate (1.0 g), Polysorbate 80 (0.5 g) and sodium chloride (0.25 g) were dissolved in water for injection. The ethanolic polymer solution was poured into the aqueous based levofloxacin solution and the total weight was added to a weight of 100.0 g. Colloidal particles precipitated as a result of this process, yielding mean particle diameters of 187.9 nm ± 1.4 with a polydispersity index of 0.14 ± 0.09. The resulting suspension was sterile filtered, using a 0.22 µm filter and 4 ml, each were filled into 5 ml amber glass vials.

### Example 6: (for comparison and/or reference only)

Gatifloxacin (8 g), Gamma-Cyclodextrin (13 g), magnesium sulfate hexahydrate (0.13 g), and sodium chloride (0.25 g) were dissolved in water for injection to a total weight of 100.0 g. The resulting solution was sterile filtered, using a 0.22 µm filter and 4 ml each, were filled under aseptic conditions into 5 ml sterile glass vials.

### Example 7: (for comparison and/or reference only)

Gatifloxacin (4 g), Amphotericin B (1g), Gamma-Cyclodextrin (8 g) and L-Arginine (0.55 g), Vitamin E-TPGS (0.1g) and sodium chloride (0.25g) were dissolved in water for injection to a total weight of 100.0 g. The resulting solution was sterile filtered, using a 0.22 µm filter and 3 ml were filled under aseptic conditions into 5 ml sterile glass vials.

### Example 8:

Levofloxacin hemihydrate (2 g), Amphotericin B (1 g), Gamma-Cyclodextrin (6 g) Chitosan (0.06 g), hydroxypropyl methylcellulose (0.20 g), magnesium sulfate hexahydrate (0.15 g), and sodium chloride (0.5 g) were dissolved in water for injection to a total weight of 100.0 g. The solution was sterile filtered, using a 0.22 µm filter and 2 ml each were filled under aseptic conditions in a laminar air flow hood into 2.5 ml preformed sterile blow fill seal vials, which were heat sealed afterwards

### Example 9: (for comparison and/or reference only)

Gatifloxacin (2 g), Ambroxol (1 g), Gamma-Cyclodextrin (5 g), magnesium sulfate hexahydrate (0.12 g), xylitol (0.5g) and sodium chloride (0.4 g) were dissolved in water for injection to a total weight of 100.0 g. The resulting solution was sterile filtered, using a 0.22 µm filter and 2 ml each, were filled under aseptic conditions into in a laminar air flow hood into 2.5 ml preformed sterile blow fill seal vials, which were heat sealed afterwards.

### Example 10: (for comparison and/or reference only)

Moxifloxacin (5 g), Fluticasone-propionate (0.02 g) Gamma-Cyclodextrin (7.5 g), magnesium sulfate hexahydrate (0.2 g), L-Arginine (0.5g) and sodium chloride (0.3 g) were dissolved in water for injection to a total weight of 100.0 g. The resulting solution was sterile filtered, using a 0.22 µm filter and 0.5 ml were filled under aseptic conditions in a laminar air flow hood into 1 ml preformed sterile blow fill seal vials, which were heat sealed afterwards.

### Example 11: (for comparison and/or reference only)

Moxifloxacin (2.5 g), Disodium cromoglycate (5 g) Gamma-Cyclodextrin (7.5 g), magnesium sulfate hexahydrate (0.5 g), L-Arginine (0.5g), saccharin sodium (0.5g) and sodium chloride (0.25 g) were dissolved in water for injection to a total weight of 100.0 g. The resulting solution was sterile filtered, using a 0.22 µm filter and 0.5 ml were filled under aseptic conditions in a laminar air flow hood into 1 ml preformed sterile blow fill seal vials, which were heat sealed afterwards.

### Cellular Tolerability Tests:

In addition to the physicochemical properties of drug formulations and their stability, either as solution or dispersed systems, it is helpful to investigate the cellular tolerability of such drug formulations in a cell culture test system. For this purpose the bronchial cell line Calu-3 was used as an in vitro model for toxicity assessment. Calu-3 represents a human submucosal adenocarcinoma cell line that forms tight, polarized and well differentiated monolayers with apical microvilli and tight junctional complexes. In contrast to other cell lines, Calu-3 cells generate extracellular mucus. Calu-3 cells have been found to be a suitable model to study the pulmonary drug delivery in vitro.

Monolayers of this cell line have been shown to have high transepithelial electrical resistances (TEER) of approximately 1000-1300 Ω·cm². In addition, it has been shown that loss of monolayer integrity is paralleled by a decrease in TEER to values below 600 Ω·cm² and an increase in permeability. Internal data show that TEER values below 200 Ω·cm² lead to an increased permeability of low permeable molecules. Since toxicity is paralleled by a loss of the monolayer integrity, a decrease in TEER of more than 50 % is indicative for the toxicity of a test compound. In general, the Calu-3 cells, which are representing bronchial cells of the upper respiratory tract, are a useful tool to study permeation of drugs as well as irritation or toxic effects of drugs, formulations and/or common excipients selected to be used in formulations for inhalation. The Calu-3 cell tests offer the possibility to investigate potential effects of the drug substance as well as the influence of excipients or formulation constituents.

The composition according to Example 1 was tested in Calu3 cells as described in detail by Keller at al. (Assessment of applicability and tolerability of drugs and excipients on Calu-3 cells, a bronchial epithelial cell model, Proceedings Drug Delivery to the Lungs XVI, Dec 7-9, 2005, pp 161-164). TEER was determined in physiological Krebs Ringer buffer (KRB) for two hours after the application of the different formulations or excipients. A decrease of the measured resistance indicated an affection of intact epithelial cell-cell contacts (tight junctions), which is an extremely sensitive parameter to determine toxic effects. To assess whether such cell damage was reversible, the cells were incubated for 24 h in cell culture subsequently to the incubation with the test formulations. On the basis of this test design differences among the formulations and excipients with a toxic potential were determined. Some of the tested excipients or formulations are listed in Table 1 below.

**Table 1**

| **Formulation** | **Dilution** / **c** | **TEER after 2 hours** | | **TEER after 24 hours** | |
|---|---|---|---|---|---|
| | | **Mean [%]** | **SD** | **Mean [%]** | **SD** |
| Negative control (KRB) p44U | - | 77 | 1 | 65 | 2 |
| Negative control | - | 85 | 1 | 83 | 1 |
| (KRB) p45U | | | | | |
| Negative control (KRB) p46U | - | 47 | 5 | 50 | 3 |
| Negative control (KRB) p47U | - | 66 | 3 | 68 | 2 |
| Positive control p44U (SDS) | 0.1% w/v in KRB | 18 | 5 | 10 | 1 |
| Positive control p45U (SDS) | 0.1% w/v in KRB | 11 | 1 | 6 | 0 |
| Positive control p46U (SDS) | 0.1% w/v in KRB | 7 | 0 | 8 | 0 |
| Positive control p47U (SDS) | 0.1% w/v in KRB | 9 | 1 | 8 | 1 |
| Captisol (100 mg·mL⁻¹) | conc. | 114 | 7 | 58 | 4 |
| | 1:5 | 98 | 1 | 56 | 4 |
| | 1:10 | 83 | 4 | 66 | 6 |
| 2-HP-β-CD (100 mg·mL⁻¹) | conc. | 59 | 7 | 45 | 10 |
| | 1:5 | 86 | 3 | 77 | 4 |
| | 1:10 | 90 | 2 | 73 | 3 |
| Gamma-CD (100 mg·mL⁻¹) | conc. | 61 | 9 | 41 | 3 |
| | 1:5 | 69 | 2 | 46 | 2 |
| | 1:10 | 65 | 10 | 44 | 2 |
| Natrium EDTA (10 mg·mL⁻¹) | conc. | 14 | 2 | 42 | 4 |
| | 1:5 | 42 | 3 | 56 | 2 |
| | 1:10 | 46 | 1 | 47 | 2 |
| Vitamin E TPGS (20 mg·mL⁻¹) | conc. | 47 | 0 | 50 | 1 |
| | 1:5 | 48 | 0 | 49 | 3 |
| | 1:10 | 47 | 0 | 51 | 1 |
| Sodium Ascorbate (20 mg·mL⁻¹) | conc. | 22 | 1 | 70 | 7 |
| | 1:5 | 70 | 0 | 66 | 2 |
| | 1:10 | 57 | 3 | 51 | 3 |
| HPMC (20 mg·mL⁻¹) | conc. | 49 | 3 | 49 | 3 |
| | 1:5 | 47 | 1 | 44 | 1 |
| Saccharin-Natrium (5 mg·mL⁻¹) | conc. | 55 | 1 | 43 | 2 |
| | 1:5 | 55 | 3 | 50 | 4 |
| Citric acid (5 mg·mL⁻¹) | conc. | 105 | 7 | 53 | 2 |
| | 1:5 | 40 | 1 | 44 | 1 |
| Amphotericin B (1 mg·mL⁻¹) | conc. | 108 | 6 | 67 | 3 |
| | 1:5 | 80 | 2 | 64 | 3 |
| Levofloxacin (25 mg/mL) | conc. | 38 | 11 | 33 | 3 |
| | 1:5 | 98 | 3 | 71 | 2 |
| Levofloxacin Placebo | conc. | 68 | 3 | 69 | 5 |
| | 1:5 | 104 | 6 | 69 | 3 |
| Tobramycin (T100) | conc. | 94 | 9 | 46 | 6 |
| | 1:5 | 123 | 4 | 79 | 4 |

It is apparent from the data summarised in Table 1 above, that the excipients proposed to soulubilise and/or complexate and/or encapsulate and/or masking unpleasant taste do not affect the vitality of Calu-3 cells. No damaging effect could be observed as well for other excipients, such as buffer salts, chelating agents, polymers (chitosan, HPMC) and surfactants and drugs, such as Tobramycin, Amphotericin B which may be advantageously combined with fluoroquinolones, such as levofloxacin. However, a direct contact of a levofloxacin formulation containing 25 mg/ ml onto the surface of Calu 3 cells, but not the placebo, affected the viability of such cells, whereas a 1 : 5 dilution is comparable to a buffer solution. It can be concluded from the results above that concentration > 25 ml / ml may be harmful to Calu-3 cells. However, it must be considered that a drug formulation will be immediately diluted upon inhalation by the epithelial lining fluid and the assessment above is only a crude indicator for the tolerability of formulations.

### Aerosolisation Experiments:

The formulation composition of Example 1 was nebulised by an eFlow electronic nebuliser (35 L configuration) generating the aerosol via a perforated vibrating mesh of orifices with a distinct diameter (R. Stangl: Development of a nebuliser product platform: Proceedings Drug Delivery to the Lungs XVI, Dec 7-9, 2005, pp 242-245). The drug delivery efficiency was assessed by breath simulation as described by Tservistas et al. (Influence of inspiratory flow rate on the delivered dose and droplet size distribution of tobramycin delivered by nebuliser systems utilizing breath simulation and the next generation impactor. Proceedings Drug Delivery to the Lungs XV, Dec 9&10, 2004, pp 220-223) and by laser diffraction regarding the droplet size distribution pattern as described by M. Knoch and E. Sommer (Jet nebuliser design and function. Eur. Respiratory Rev. 2000; 10: 72, 183-186). The Results are shown in Table 2.

**Table 2**

| Breathing pattern | Tidal Volume 200ml; 25 bpm; inh:exh. = 1:1 | | Tidal Volume 500ml; 15 bpm; inh:exh. = 1:1 | | Tidal Volume 1000 ml; 25 bpm; inh:exh. = 4:6 | |
|---|---|---|---|---|---|---|
| Nebulizer system | eFlow | | eFlow | | eFlow | |
| | Mean | SD | Mean | SD | Mean | SD |
| Delivered Dose [%] | **69.7** | 2.5 | **67.1** | 1.7 | **70.3** | 2.2 |
| Aerosol Losses [%] | **16.7** | 1.3 | **17.7** | 0.6 | **19.2** | 7.0 |
| Drug residue [%] | **8.7** | 3.0 | **9.6** | 0.9 | **7.5** | 0.6 |
| Nebulization Time | **2.96** | 0.44 | **3.27** | 0.57 | **2.98** | 0.32 |

Table 2 shows the delivery efficiency upon nebulisation of 1.5 ml Levofloxacin (25 mg/ml) formulation according to example 1 by three eFlow electronic nebulisers, each. Results are the mean of 6 experiments conducted in duplicate using three eFlow electronic nebulisers. It is apparent from the results in the Table 2 above, that a Levofloxacin inhalation solution containing 25 mg/ml according to a composition as outlined in Example 1 can be very efficiently nebulised by eFlow. Surprisingly, the delivery performance is nearly independent of the breathing pattern applied and neither the tidal volume nor the number of breaths nor the inhalation to exhalation ratio has a significant effect on the delivered dose ranging from 67.1 - 70.3 % of the total loaded Levofloxacin dose (36 mg). Hence, eFlow is better suited compared to jet nebulisers, known to have a flow dependent delivery performance (M. Tservistas et al: Proceedings Drug Delivery to the Lungs XV, Dec 9&10, 2004, pp 220-223) to deliver Fluoroquinolone to the lungs. Furthermore, the short nebulisation time of about 3 min for a 1.5 ml volume should help to improve patients compliance.

## Claims

1. A pharmaceutical aerosol for nasal, sinunasal or pulmonary administration comprising a dispersed liquid phase and a continuous gas phase, wherein the dispersed liquid phase
(a) consists of aqueous droplets comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts;
(b) has a mass median diameter from about 1.5 to about 6 µm; and
(c) has a droplet size distribution exhibiting a geometrical standard deviation from about 1.2 to about 3.0.

2. The aerosol of claim 1, wherein the at least one excipient is selected from the group of magnesium salts.

3. The aerosol of any of claims 1 to 2, being emitted from an aerosol generator at a rate of at least about 0.1 ml dispersed liquid phase per minute.

4. A liquid pharmaceutical composition for preparing the aerosol of any one of claims 1 to 3, wherein the composition comprises 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and wherein a volume of not more than about 10 ml and more preferably less than about 5 ml of the composition comprises an effective dose of the active compound.

5. The liquid pharmaceutical composition of claim 4, wherein the at least one excipient is selected from the group of magnesium salts.

6. The composition of any of claims 4 to 5, comprising at least one excipient capable of affecting the local bioavailability, the release, and/or the local residence time of the active compound at the site of aerosol deposition, wherein the excipient is selected from the group consisting of complexing agents, polymers, and amphiphilic compounds.

7. The composition of any one of claims 4 to 6, comprising at least one taste-modifying excipient, preferably selected from flavours, sweeteners, complexing agents and taste masking agents, such as a cyclodextrin, sugar, sugar alcohol, saccharin sodium, aspartame, or arginine.

8. A kit for the preparation and delivery of a pharmaceutical aerosol for nasal, sinunasal or pulmonary administration comprising a dispersed liquid phase and a continuous gas phase, wherein the dispersed liquid phase
(a) consists of aqueous droplets comprising 20 to 200 mg/ml of the active compound levofloxacin or the equivalent concentration of a pharmaceutically acceptable salt, solvate or isomer thereof and at least one excipient selected from the group of calcium-, magnesium-, and aluminum salts;
(b) has a mass median diameter from about 1.5 to about 6 µm; and
(c) has a droplet size distribution exhibiting a geometrical standard deviation from about 1.2 to about 3.0,
wherein the kit comprises a nebuliser and an aqueous liquid composition, said composition comprising 20 to 200 mg/ml of the active compound levofloxacin or the equivalent concentration of a pharmaceutically acceptable salt, solvate or isomer thereof, and at least one excipient selected from the group of calcium-, magnesium-, and aluminum salts, and comprising an effective dose of the active compound within a volume of not more than about 10 ml and more preferably less than 5 ml.

9. The kit of claim 8, wherein the at least one excipient is selected from the group of magnesium salts.

10. The kit of any of claims 8 to 9, wherein at least about 40 wt.-% of the loaded dose is comprised of droplets having a diameter of not more than about 5 µm.

11. A method of preparing an aerosol for delivery to a person in need of nasal, sinunasal or pulmonary antibiotic treatment or prophylaxis, said method comprising the steps of:
(a) providing a liquid pharmaceutical composition comprising 20 to 200 mg/ml of the active compound levofloxacin or the equivalent concentration of a pharmaceutically acceptable salt, solvate or isomer thereof and at least one excipient selected from the group of calcium-, magnesium-, and aluminum salts, and comprising an effective dose of the active compound in a volume of not more than about 10 ml and more preferably less than 5 ml;
(b) providing a nebuliser capable of aerosolising said liquid pharmaceutical composition at a total output rate of at least 0.1 ml/min, the nebuliser further being adapted to emit an aerosol comprising a dispersed phase having a mass median diameter from about 1.5 to about 6 µm and a geometrical standard deviation from about 1.2 to about 3; and
(c) operating the nebuliser to aerosolise the liquid composition.

12. The method of claim 11, wherein the at least one excipient is selected from the group of magnesium salts.

13. Use of the aerosol of claim 1 or of the liquid composition of claim 4 or of the kit of claim 8 for the preparation of a medicament for the prophylaxis or treatment of acute or chronic sinusitis or rhinosinusitis, bronchitis, pneumonia, chronic obstructive pulmonary disease, prophylaxis to prevent graft rejection after lung transplantation, parenchymatic and/or fibrotic diseases or disorders including cystic fibrosis with or without acute exacerbations, optionally due to Streptococcus pneumoniae, Haemophilus influenza or Moraxella catarrhalis; acute bacterial exacerbations in chronic bronchitis or in chronic obstructive pulmonary disease, optionally due to Staphylococcus aureus, Streptococcus pneumoniae, Haemophilus influenza, Haemophilus parainfluenza or Moraxella catarrhalis; nosocomial pneumonia, optionally due to Staphylococcus aureus, Pseudomonas aeruginosa, Serratia marcescens, Burkholderia cepacia, Escherichia coli, Klebsiella pneumoniae, Haemophilus influenza or Streptococcus pneumoniae; or community acquired pneumonia (CAP), or hospital acquired pneumonia (HAP), or ventilator associated pneumonia (VAP), optionally due to Staphylococcus aureus, Streptococcus pneumoniae, Haemophilus influenza, Haemophilus parainfluenza, Klebsiella pneumoniae, Moraxella catarrhalis, Chlamydia pneumoniae, Legionella pneumophila, or Mycoplasma pneumoniae.

14. The use of claim 13, wherein the medicament is administered pulmonary.

15. The use of any of claims 13 to 14, wherein the medicament is adapted for twice or once daily administration.

16. The use of a polymeric compound as excipient in a pharmaceutical composition for the preparation of an aerosol, wherein the composition comprises 20 to 200 mg/ml of the active compound levofloxacin or the equivalent concentration of a pharmaceutically acceptable salt, solvate or isomer thereof, and at least one excipient selected from the group ofcalcium-, magnesium-, and aluminum salts, and wherein the polymeric compound is selected from chitosan.

## Patentansprüche

1. Pharmazeutisches Aerosol für die nasale, sinunasale oder pulmonale Verabreichung, umfassend eine dispergierte flüssige Phase und eine kontinuierliche Gasphase, wobei die dispergierte flüssige Phase
(a) besteht aus wässrigen Tröpfchen, umfassend 20 bis 200 mg/ml der aktiven Verbindung Levofloxacin oder die äquivalente Konzentration eines pharmazeutisch annehmbaren Salzes, Solvats oder Isomers davon und mindestens einen Hilfsstoff, ausgewählt aus der Gruppe von Calcium-, Magnesium- und Aluminiumsalzen;
(b) einen Massen-bezogenen medianen Durchmesser von etwa 1,5 bis etwa 6 µm hat; und
(c) eine Tröpfchengrößenverteilung hat, die eine geometrische Standardabweichung von etwa 1,2 bis etwa 3,0 aufweist.

2. Aerosol gemäß Anspruch 1, wobei der mindestens eine Hilfsstoff ausgewählt ist aus der Gruppe von Magnesiumsalzen.

3. Aerosol gemäß einem der Ansprüche 1 bis 2, das von einem Aerosolgenerator mit einer Geschwindigkeit von mindestens etwa 0,1 ml dispergierte flüssige Phase pro Minute emittiert wird.

4. Flüssige pharmazeutische Zusammensetzung zur Herstellung des Aerosols gemäß einem der Ansprüche 1 bis 3, wobei die Zusammensetzung 20 bis 200 mg/ml der aktiven Verbindung Levofloxacin oder die äquivalente Konzentration eines pharmazeutisch annehmbaren Salzes, Solvats oder Isomers davon enthält und mindestens einen Hilfsstoff, ausgewählt aus der Gruppe von Calcium-, Magnesium- und Aluminiumsalzen, und wobei ein Volumen von nicht mehr als etwa 10 ml und mehr bevorzugt weniger als 5 ml der Zusammensetzung eine wirksame Dosis der aktiven Verbindung enthält.

5. Flüssige pharmazeutische Zusammensetzung gemäß Anspruch 4, wobei der mindestens eine Hilfsstoff ausgewählt ist aus der Gruppe von Magnesiumsalzen.

6. Zusammensetzung gemäß einem der Ansprüche 4 bis 5, umfassend mindestens einen Hilfsstoff, der die lokale Bioverfügbarkeit, die Freisetzung und/oder die lokale Verweilzeit der aktiven Verbindung an der Stelle der Aerosoldeposition beeinflussen kann, wobei der Hilfsstoff ausgewählt ist aus der Gruppe, bestehend aus Komplexbildnern, Polymeren und amphiphilen Verbindungen.

7. Zusammensetzung gemäß einem der Ansprüchen 4 bis 6, umfassend mindestens einen geschmacksmodifizierenden Hilfsstoff, bevorzugt ausgewählt aus Aromen, Süßstoffen, Komplexbildnern und Geschmacksmaskierungsmitteln, wie ein Cyclodextrin, Zucker, Zuckeralkohol, Natriumsaccharin, Aspartam oder Arginin.

8. Kit zur Herstellung und Verabreichung eines pharmazeutischen Aerosols für die nasale, sinunasale oder pulmonale Verabreichung, umfassend eine dispergierte flüssige Phase und eine kontinuierliches Gasphase, wobei die dispergierte flüssige Phase
(a) besteht aus wässrigen Tröpfchen, umfassend 20 bis 200 mg/ml der aktiven Verbindung Levofloxacin oder die äquivalente Konzentration eines pharmazeutisch annehmbaren Salzes, Solvats oder Isomers davon und mindestens einen Hilfsstoff, ausgewählt aus der Gruppe von Calcium-, Magnesium- und Aluminiumsalzen;
(b) einen Massen-bezogenen medianen Durchmesser von etwa 1,5 bis etwa 6 µm hat; und
(c) eine Tröpfchengrößenverteilung hat, die eine geometrische Standardabweichung von etwa 1,2 bis etwa 3,0 aufweist, wobei das Kit einen Vernebler und eine wässrige flüssige Zusammensetzung umfasst, wobei die Zusammensetzung 20 bis 200 mg/ml der aktiven Verbindung Levofloxacin oder die äquivalente Konzentration eines pharmazeutisch annehmbaren Salzes, Solvats oder Isomers davon enthält, und mindestens ein Hilfsstoff, ausgewählt aus der Gruppe von Calcium-, Magnesium- und Aluminiumsalzen, und eine wirksame Dosis der aktiven Verbindung in einem Volumen von nicht mehr als etwa 10 ml und mehr bevorzugt weniger als 5 ml enthält.

9. Kit gemäß Anspruch 8, wobei der mindestens eine Hilfsstoff ausgewählt ist aus der Gruppe von Magnesiumsalzen.

10. Kit gemäß einem der Ansprüche 8 bis 9, wobei mindestens etwa 40 Gewichtsprozent der eingefüllten Dosis Tröpfchen mit einem Durchmesser von nicht mehr als etwa 5 µm enthält.

11. Verfahren zur Herstellung eines Aerosols zur Verabreichung an eine Person, die eine nasale, sinunasale oder pulmonale antibiotische Behandlung oder Prophylaxe benötigt, wobei das Verfahren die folgenden Schritte umfasst:
(a) Bereitstellen einer flüssigen pharmazeutischen Zusammensetzung, umfassend 20 bis 200 mg/ml der aktiven Verbindung Levofloxacin oder die äquivalente Konzentration eines pharmazeutisch annehmbaren Salzes, Solvats oder Isomers davon und mindestens einen Hilfsstoff, ausgewählt aus der Gruppe von Calcium-, Magnesium- und Aluminiumsalzen, und umfassend eine wirksame Dosis der aktiven Verbindung in einem Volumen von nicht mehr als etwa 10 ml und mehr bevorzugt weniger als 5 ml;
(b) Bereitstellen eines Verneblers, der die flüssige pharmazeutische Zusammensetzung mit einer Gesamtausgabegeschwindigkeit von mindestens 0,1 ml/min aerosolisieren kann, wobei der Vernebler weiter zum Emittieren eines Aerosols, umfassend eine dispergierte Phase mit einem Massen-bezogenen medianen Durchmesser von etwa 1,5 bis etwa 6 µm und einer geometrischen Standardabweichung von etwa 1,2 bis etwa 3, angepasst ist; und
(c) Betätigen des Verneblers zum Aerosolisieren der flüssigen Zusammensetzung.

12. Verfahren gemäß Anspruch 11, wobei der mindestens eine Hilfsstoff ausgewählt ist aus der Gruppe von Magnesiumsalzen.

13. Verwendung des Aerosols gemäß Anspruch 1 oder der flüssigen Zusammensetzung gemäß Anspruch 4 oder des Kits gemäß Anspruch 8 zur Herstellung eines Arzneimittels für die Prophylaxe oder Behandlung von akuter oder chronischer Sinusitis oder Rhinosinusitis, Bronchitis, Lungenentzündung, chronisch obstruktiver Lungenerkrankung, Prophylaxe zum Verhindern einer Transplantationsabstoßung nach Lungentransplantation, parenchymatöser und/oder fibrotischer Erkrankung oder Störungen einschließlich zystischer Fibrose mit oder ohne akute(n) Exazerbationen, gegebenenfalls verursacht durch Streptococcus pneumoniae, Haemophilus influenza oder Moraxella catarrhalis; akuter bakterieller Exazerbationen bei chronischer Bronchitis oder bei chronisch obstruktiver Lungenerkrankung, gegebenenfalls verursacht durch Staphylococcus aureus, Streptococcus pneumoniae, Haemophilus influenza, Haemophilus parainfluenza oder Moraxella catarrhalis; nosokomialer Lungenentzündung, gegebenenfalls verursacht durch Staphylococcus aureus, Pseudomonas aeruginos, Serratia marcescens, Burkholderia cepacia, Escherichia coli, Klebsiella pneumoniae, Haemophilus influenza oder Streptococcus pneumoniae; oder ambulanterworbener Lungenentzündung (community acquired pneumonia, CAP) oder im Krankenhaus erworbener Lungenentzündung (hospital acquired pneumonia, HAP) oder mit Beatmung verbundener Lungenentzündung (ventilator associated pneumonia, VAP), gegebenenfalls verursacht durch Staphylococcus aureus, Streptococcus pneumoniae, Haemophilus influenza, Haemophilus parainfluenza, Klebsiella pneumoniae Moraxella catarrhalis, Chlamydia pneumoniae, Legionella pneumophila oder Mycoplasma pneumoniae.

14. Verwendung gemäß Anspruch 13, wobei das Arzneimittel pulmonal verabreicht wird.

15. Verwendung gemäß einem der Ansprüche 13 bis 14, wobei das Arzneimittel für die zweimalige oder einmalige Verabreichung pro Tag angepasst ist.

16. Verwendung einer polymeren Verbindung als Hilfsstoff einer pharmazeutischen Zusammensetzung für die Herstellung eines Aerosols, wobei die Zusammensetzung 20 bis 200 mg/ml der aktiven Verbindung Levofloxacin oder eine äquivalente Konzentration eines pharmazeutisch annehmbaren Salzes, Solvats oder Isomers davon enthält und mindestens ein Hilfsstoff, ausgewählt aus der Gruppe von Calcium-, Magnesium- und Aluminiumsalzen, und wobei die polymere Verbindung ausgewählt ist aus Chitosan.

## Revendications

1. Un aérosol pharmaceutique pour l'administration nasale, sinusnasale ou pulmonaire, comprenant une phase liquide dispersée et une phase gazeuse continue, dans lequel la phase liquide dispersée
(a) consiste essentiellement en gouttelettes aqueuses comprenant de 20 à 200 mg/ml du composé actif lévofloxacine ou la concentration équivalente d'un sel pharmaceutiquement acceptable, d'un solvate pharmaceutiquement acceptable ou d'un isomère pharmaceutiquement acceptable de celui-ci et au moins un excipient choisi dans le groupe des sels de calcium, magnésium et aluminium;
(b) a un diamètre médian en masse d'environ 1,5 à environ 6 µm, et
(c) a une distribution de taille de gouttelettes présentant un écart type géométrique d'environ 1,2 à environ 3,0.

2. L'aérosol selon la revendication 1, dans lequel l'au moins un excipient est choisi dans le groupe des sels de magnésium.

3. L'aérosol selon l'une quelconque des revendications 1 à 2 qui est émis par un générateur d'aérosol à raison d'au moins environ 0,1 ml de phase liquide dispersée par minute.

4. Une composition pharmaceutiquement liquide pour la préparation de l'aérosol selon l'une quelconque des revendications 1 à 3, la composition comprenant: de 20 à 200 mg/ml du composé actif lévofloxacine ou la concentration équivalente d'un sel pharmaceutiquement acceptable, d'un solvate pharmaceutiquement acceptable ou d'un isomère pharmaceutiquement acceptable de celui-ci et au moins un excipient choisi dans le groupe des sels de calcium, magnésium et aluminium, et dans laquelle un volume non supérieur à environ 10 ml et plus préférablement inférieur à environ 5 ml de la composition comprend une dose efficace du composé actif.

5. La composition pharmaceutiquement liquide selon la revendication 4, dans laquelle l'au moins un excipient est choisi dans le groupe des sels de magnésium.

6. La composition selon l'une quelconque des revendications 4 à 5, comprenant au moins un excipient susceptible d'affecter la biodisponibilité, la libération et/ou le temps de séjour local du composé actif au site de dépôt par aérosol, dans laquelle ledit excipient est choisi dans le groupe constitué par des agents complexants, des polymères et des composés amphiphiles.

7. La composition selon l'une quelconque des revendications 4 à 6, comprenant au moins un excipient modifiant le goût, de préférence choisi parmi des arômes, des édulcorants, des agents complexants et des agents masquant le goût, tels qu'une cyclodextrine, un sucre, un alcool de sucre, la saccharine sodique, l'aspartame ou l'arginine.

8. Une trousse pour la préparation et la délivrance d'un aérosol pharmaceutiquement pour l'administration nasale, sinusnasale ou pulmonaire, comprenant une phase liquide dispersée et une phase gazeuse continue, dans lequel la phase liquide dispersée
(a) consiste essentiellement en gouttelettes aqueuses comprenant de 20 à 200 mg/ml du composé actif lévofloxacine ou la concentration équivalente d'un sel pharmaceutiquement acceptable, d'un solvate pharmaceutiquement acceptable ou d'un isomère pharmaceutiquement acceptable de celui-ci et au moins un excipient choisi dans le groupe des sels de calcium, magnésium et aluminium;
(b) a un diamètre médian en masse d'environ 1,5 à environ 6 µm; et
(c) a une distribution de taille de gouttelettes présentant un écart type géométrique d'environ 1,2 à environ 3,0, dans laquelle la trousse comprend un nébuliseur et une composition liquide aqueuse, ladite composition comprenant: de 20 à 200 mg/ml du composé actif lévofloxacine ou la concentration équivalente d'un sel pharmaceutiquement acceptable, d'un solvate pharmaceutiquement acceptable ou d'un isomère pharmaceutiquement acceptable de celui-ci et au moins un excipient choisi dans le groupe des sels de calcium, magnésium et aluminium, et dans laquelle un volume non supérieur à environ 10 ml et plus préférablement inférieur à environ 5 ml de la composition comprend une dose efficace du composé actif.

9. La trousse selon la revendication 8, dans laquelle l'au moins un excipient est choisi dans le groupe des sels de magnésium.

10. La trousse selon l'une quelconque des revendications 8 à 9, dans laquelle au moins environ 40% en poids de la dose chargée sont constitués de gouttelettes ayant un diamètre non supérieur à environ 5 µm.

11. Un procédé de préparation d'un aérosol pour la délivrance à une personne nécessitant un traitement antibiotique par voie nasale, sinusnasale ou pulmonaire ou une prophylaxie, ledit procédé comprenant les étapes consistant à:
(a) fournir une composition pharmaceutique liquide, comprenant de 20 à 200 mg/ml du composé actif lévofloxacine ou la concentration équivalente d'un sel pharmaceutiquement acceptable, d'un solvate pharmaceutiquement acceptable ou d'un isomère pharmaceutiquement acceptable de celui-ci et au moins un excipient choisi dans le groupe des sels de calcium, magnésium et aluminium, et comprenant une dose efficace du composé actif dans un volume non supérieure à environ 10 ml et plus préférablement inférieur à environ 5 ml;
(b) fournir un nébuliseur capable d'aérosoliser ladite composition pharmaceutique liquide à une vitesse de sortie totale d'au moins 0,1 ml/min, le nébuliseur étant en outre adapté pour émettre un aérosol comprenant une phase dispersée ayant un diamètre médian en masse d'environ 1,5 à environ 6 µm et un écart type géométrique d'environ 1,2 à environ 3,0; et
(c) faire fonctionner le nébuliseur pour aérosoliser la composition liquide.

12. Le procédé selon la revendication 11, dans lequel l'au moins un excipient est choisi dans le groupe des sels de magnésium.

13. Une utilisation de l'aérosol de la revendication 1 ou de la composition liquide de la revendication 4 ou de la trousse de la revendication 8 pour la préparation d'un médicament pour la prophylaxie ou le traitement de la sinusite aiguë ou chronique, de la bronchite, de la pneumonie, de la broncho-pneumopathie chronique obstructive, pour la prophylaxie pour prévenir le rejet de greffe après une transplantation pulmonaire, des maladies ou des troubles parenchymateux et/ou fibrotiques comprenant la fibrose kystique avec ou sans exacerbations aiguës, facultativement due à Streptococcus pneumoniae, Haemophilus influenza ou Moraxella catarrhalis; des exacerbations bactériennes aiguës dans la bronchite chronique ou dans la maladie pulmonaire obstructive chronique, éventuellement dues à Staphylococcus aureus, Streptococcus pneumoniae, Haemophilus influenza, Haemophilus parainfluenza ou Moraxella catarrhalis; la pneumonie nosocomiale, éventuellement due à Staphylococcus aureus, à Pseudomonas aeruginosa, à Serratia marcescens, à Burkholderia cepacia, à Escherichia coli, à Klebsiella pneumoniae, à Haemophilus influenza ou à Streptococcus pneumoniae; ou une pneumonie acquise dans la communauté (CAP), ou une pneumonie acquise à l'hôpital (HAP), ou une pneumonie associée à la ventilation (VAP), éventuellement due à Staphylococcus aureus, Streptococcus pneumoniae, Haemophilus influenzae, Haemophilus parainfluenza, Klebsiella pneumoniae, Moraxella catarrhalis, Chlamydia pneumoniae, Legionella pneumophila ou Mycoplasma pneumoniae.

14. L'utilisation selon la revendication 13, dans laquelle le médicament est administré par voie pulmonaire.

15. L'utilisation selon l'une quelconque des revendications 13 à 14, dans laquelle le médicament est adapté pour une administration deux fois ou une fois par jour.

16. Une utilisation d'un composé polymère en tant qu'excipient dans une composition pharmaceutique pour la préparation d'un aérosol, la composition comprenant 20 à 200 mg/ml du composé actif lévofloxacine ou la concentration équivalente d'un sel pharmaceutiquement acceptable ou d'un solvate pharmaceutiquement acceptable ou d'un isomère pharmaceutiquement acceptable de celui-ci et au moins un excipient choisi dans le groupe des sels de calcium, magnésium et aluminium, et dans laquelle le composé polymère est choisi parmi chitosan.
